# EUROPEAN PATENT APPLICATION

(11) **EP 4 386 694 A1**
(43) Date of publication of application: **19.06.2024**
(21) Application number: 23213578.0
(22) Date of filing: 01.12.2023
(51) Int. Cl.: G06V 10/82, G01N 23/2251, G01N 33/44, G06N 3/045, G06V 20/69

(54) **ANALYSIS METHOD FOR RUBBER COMPOSITION AND GENERATION METHOD FOR TRAINED MODEL**

(30) Priority: 13.12.2022 JP 2022198564
(71) Applicant: Sumitomo Rubber Industries, Ltd., Kobe-shi, Hyogo 651-0072 (JP); NEC Corporation, Tokyo 108-8001 (JP)
(72) Inventor: ITO, Wakana, Kobe-shi, Hyogo, 651-0072 (JP); KONDO, Takashi, Tokyo, 108-8001 (JP); ISHIZAWA, Yoshio, Tokyo, 108-8001 (JP); SAKAMOTO, Naoto, Tokyo, 108-8001 (JP); NAKAMURA, Hiroki, Tokyo, 108-8001 (JP)
(74) Representative: Manitz Finsterwald Patent- und Rechtsanwaltspartnerschaft mbB

(57) **Abstract**

Provided is an analysis method and the like for appropriately defining a region corresponding to a formulation contained in a rubber composition in a microscopic image of the rubber composition. An analysis method for a rubber composition includes the followings: acquiring input data generated from a microscopic image obtained by image-capturing a rubber composition with a microscope, the microscopic image showing a formulation contained in the rubber composition; inputting the input data to a trained machine learning model; and deriving output data from the trained machine learning model. Note that the output data is data defining a region that appears in the microscopic image corresponding to the formulation.

## Description

### FIELD OF INVENTION

The present invention relates to an analysis method for a rubber composition and a generation method for a trained model.

### BACKGROUND

Conventionally, in order to deepen knowledge about a rubber composition, there has been provided a technique for image-capturing the rubber composition with a microscope and analyzing the obtained microscopic image. For example, JP 2019-021037 A discloses a technique of calculating, from a microscopic image of a rubber composition, an index indicating a feature of the microscopic image, and estimating a characteristic of the rubber composition based on the index.

### SUMMARY of INVENTION

In the technique disclosed in JP 2019-021037 A, a feature vector calculated from the microscopic image of the rubber composition is associated with a specific characteristic of the rubber composition. On the other hand, in the microscopic image of the rubber composition, the formulation contained in the rubber composition appears as a difference in brightness, and in order to extract quantitative data on the physical properties and structure of the rubber composition, it may be desired to first distinguish a region corresponding to a specific formulation. However, it is not easy to properly define such a region in the microscopic image of the rubber composition, and such a technique has not been provided so far.

An object of the present invention is to provide an analysis method for a rubber composition and a generation method for a trained model for appropriately defining a region corresponding to a formulation contained in a rubber composition in a microscopic image of the rubber composition.

An analysis method for a rubber composition according to a first aspect includes the followings:
(1) acquiring input data generated from a microscopic image obtained by image-capturing a rubber composition with a microscope, the microscopic image showing a formulation contained in the rubber composition;
(2) inputting the input data to a trained machine learning model; and
(3) deriving output data from the trained machine learning model.

Note that the output data is data defining a region that appears in the microscopic image corresponding to the formulation.

An analysis method according to a second aspect is the analysis method according to the first aspect, further including deriving a feature amount related to at least one of a structure and a physical property of the rubber composition based on the output data.

An analysis method according to a third aspect is the analysis method according to the first aspect or the second aspect, in which the trained machine learning model is configured as a segmentation model.

An analysis method according to a fourth aspect is the analysis method according to any one of the first to third aspects, in which the trained machine learning model is configured as a model selected from a group consisting of an attention network, SegNet, a feature pyramid network, UNet, PSPNet, TransNet, and TransUNet, or a model based on a model selected from the group.

An analysis method according to a fifth aspect is the analysis method according to any one of the first to fourth aspects, in which the trained machine learning model is configured as a model including a Transformer encoder and a UNet encoder.

An analysis method according to a sixth aspect is the analysis method according to the fifth aspect, in which inputting the input data to the trained machine learning model includes inputting data based on the input data to the Transformer encoder and inputting the input data to the UNet encoder.

An analysis method according to a seventh aspect is the analysis method according to any one of the first to sixth aspects, in which the microscope is a backscattered electron microscope.

An analysis method according to an eighth aspect is the analysis method according to the second aspect, in which the feature amount relates to at least one of a dispersion state of the formulation, a shape of texture formed by the formulation, and a size of texture formed by the formulation.

An analysis method according to a ninth aspect is the analysis method according to the second aspect, in which the feature amount relates to at least one of viscosity, hardness, toluene swelling index, specific gravity, glass transition temperature (Tg), temperature dispersion (TD), modulus, tensile strength, elongation, storage elastic modulus, and loss elastic modulus of the rubber composition.

A generation method for a trained model according to a tenth aspect includes the followings:
(1) preparing learning data in which a plurality of pieces of first data generated from a microscopic image obtained by image-capturing a rubber composition with a microscope, the microscopic image showing a formulation contained in the rubber composition, and a plurality of pieces of second data defining a region appearing in the microscopic image corresponding to the formulation are combined;
(2) dividing the learning data into training data and verification data; and
(3) adjusting a parameter defining a machine learning model such that when data corresponding to the first data is input using the training data, data corresponding to the second data is output.

A generation method for a trained model according to an eleventh aspect is the generation method for a trained model according to the tenth aspect, and further includes the followings:
(4) deriving output data corresponding to the second data by inputting the first data included in the verification data to the machine learning model in which the parameter is adjusted;
(5) calculating an error of the output data with respect to the second data; and
(6) specifying the first data included in the verification data in which the error is relatively small.

A generation method for a trained model according to a twelfth aspect is the generation method for generating a trained model according to the eleventh aspect, and further includes the following:
(7) newly preparing similar data having a high degree of similarity to the specified first data;
(8) deriving output data by inputting the similar data to the machine learning model in which the parameter is adjusted;
(9) using the derived output data as pseudo correct answer data, creating pseudo learning data in which the similar data and the pseudo correct answer data are combined; and
(10) further adjusting the parameter such that when the similar data is input to the machine learning model in which the parameter is adjusted, data corresponding to the pseudo correct answer data is output.

An analysis device according to a thirteenth aspect includes one or a plurality of processors configured to execute the analysis method according to any one of the first to ninth aspects. Furthermore, an analysis program according to a fifteenth aspect causes one or a plurality of processors to execute the analysis method according to any one of the first to ninth aspects.

A generation device for a trained model according to a fourteenth aspect includes one or a plurality of processors configured to execute the generation method for a trained model according to any one of the tenth to twelfth aspects. Furthermore, a generation program for a trained model according to a sixteenth aspect causes one or a plurality of processors to execute the generation method for a trained model according to any one of the tenth to the twelfth aspects.

According to the present invention, in a microscopic image obtained by image-capturing a rubber composition with a microscope, a region corresponding to a formulation contained in the rubber composition is defined by a trained machine learning model. This makes it possible to perform region division that is difficult with an image processing algorithm that does not include machine learning, and consequently, to deepen knowledge about at least one of the structure and physical properties of the rubber composition.

### BRIEF DESCRIPTION OF DRAWINGS

FIG. 1A is an example of a microscopic image obtained by image-capturing a rubber composition with an electron microscope;
FIG. 1B is an example of a microscopic image obtained by image-capturing the rubber composition with an electron microscope;
FIG. 1C is an example of an image obtained by performing region division by conventional image processing;
FIG. 2 is a block diagram illustrating an electrical configuration of an analysis device according to an embodiment;
FIG. 3 is a diagram for describing a structure of a machine learning model according to an embodiment;
FIG. 4 is a flowchart showing a flow of an analysis method by the analysis device;
FIG. 5 is a diagram illustrating a principle of deriving a feature amount according to an embodiment;
FIG. 6 is a diagram illustrating a principle of deriving a feature amount according to an embodiment;
FIG. 7 is an example of a histogram generated based on FIGS. 5 and 6;
FIG. 8 is a diagram for describing features extracted from positions of plot points on a histogram;
FIG. 9 is a flowchart illustrating a flow of a generation method for a machine learning model;
FIG. 10A is a diagram illustrating an accuracy index of a model according to an example;
FIG. 10B is a diagram illustrating an accuracy index of a model according to another example;
FIG. 11 is a diagram for comparing input data, correct answer data, and output data;
FIG. 12 is a diagram for comparing microscopic images of different formulations with region divided images; and
FIG. 13 is a diagram visualizing a correlation between a feature amount of a formulation and physical properties.

### DETAILED DESCRIPTION OF THE PREFERRED EMBODIMENTS

Hereinafter, an analysis method for a rubber composition executed by an analysis device according to an embodiment and a generation method for a trained model executed by a generation device for a trained model according to an embodiment will be described.

### <1. Overview>

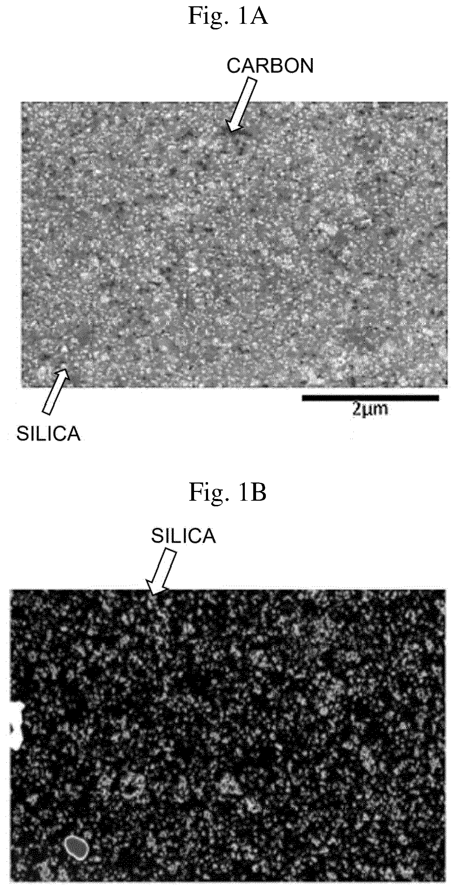

A rubber composition is a polymer compound having elasticity, and is typically generated by kneading a plurality of formulations together. Examples of the type of the formulation include a polymer, a filler (silica, carbon, etc.), and a crosslinking agent. Here, the rubber composition may be one before crosslinking or one after crosslinking. In a microscopic image obtained by image-capturing such a rubber composition with a microscope, at least a part of the formulation appears as a difference in brightness of each pixel. Both FIGS. 1A and 1B are examples of such microscopic images. FIG. 1A is an example of a microscopic image obtained by image-capturing a rubber composition with a backscattered electron microscope, and FIG. 1B is an example of a microscopic image obtained by image-capturing a rubber composition with a secondary electron microscope. In FIG. 1A, a region of relatively high brightness corresponds to an aggregate of silica, a region of relatively low brightness corresponds to an aggregate of carbon, and a region of intermediate brightness corresponds to one or a plurality of polymers. In FIG. 1B, a region having a relatively high brightness corresponds to an aggregate of silica. As described above, in the microscopic image of the rubber composition, information mainly related to a structure of the rubber composition, such as a shape and a size of a texture formed by the formulation, and a distance between the textures (a dispersion state of the formulation), appears.

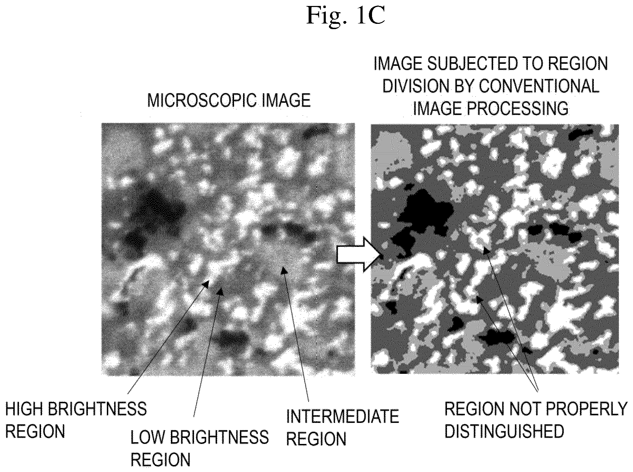

In order to extract the above information as quantitative data, it is necessary to define a region corresponding to at least one type of formulation on the microscopic image and to divide it from the other regions. However, according to a known image processing algorithm that divides the region based on the brightness of each pixel of the microscopic image, it is difficult to perform region division according to conventional knowledge on the rubber composition. For example, it is assumed that, in one microscopic image, there are a high brightness region having a relatively high brightness and corresponding to a first formulation, a low brightness region having a relatively low brightness and corresponding to a second formulation, and an intermediate region having an intermediate brightness range and corresponding to a third formulation. In such a case, a known image processing algorithm cannot distinguish between a boundary region present between the high brightness region and the low brightness region and the intermediate region corresponding to the third formulation, which may result in a region division result that does not match the actual state of the formulations (see FIG. 1C).

In view of such a problem, an analysis device 1 is configured to execute region division on a microscopic image of a rubber composition using a trained machine learning model 131 (hereinafter, also simply referred to as "model 131") to be described later. In addition, the analysis device 1 of the present embodiment is also configured as a generation device for a trained model that causes a machine learning model to learn and generates the model 131. Hereinafter, the configuration of the analysis device 1 will be described.

### <2. Configuration of analysis device>

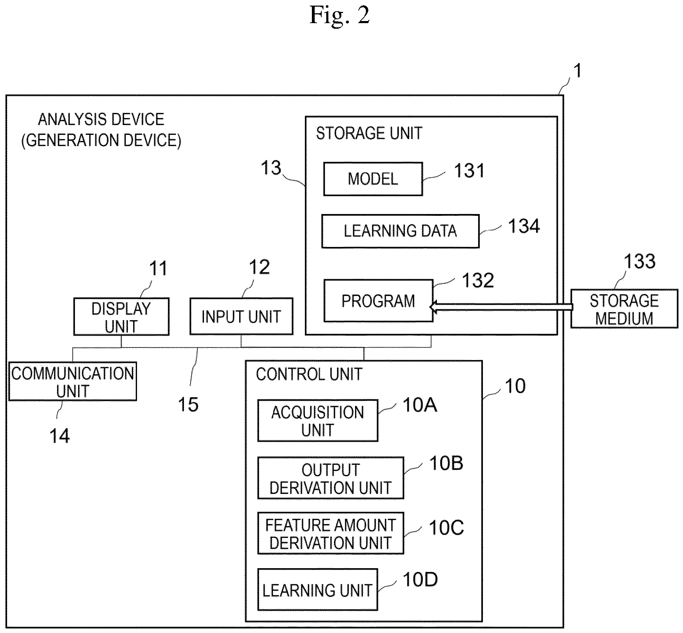

FIG. 2 is a block diagram illustrating an electrical configuration of the analysis device 1. The analysis device 1 is a general-purpose computer as hardware, and is realized as an information processing device such as a desktop personal computer, a laptop personal computer, a tablet, or a smartphone. The analysis device 1 is manufactured by installing a program 132 in a general-purpose computer from a computer-readable storage medium 133 such as a CD-ROM or a USB memory or via a network. The program 132 causes the analysis device 1 to execute an operation described later.

The analysis device 1 includes a control unit 10, a display unit 11, an input unit 12, a storage unit 13, and a communication unit 14. These units 10 to 14 are connected to each other via a bus line 15 and can communicate with each other. At least a part of the display unit 11, the input unit 12, and the storage unit 13 may be integrally incorporated in a main body (a housing that houses the control unit 10 and the like) of the analysis device 1 or may be externally attached.

The display unit 11 can include a liquid crystal display, an organic EL display, a plasma display, a touch panel display, or the like, and displays various screens generated by the control unit 10. The input unit 12 can include a mouse, a keyboard, a touch panel, or the like, and receives a user's operation on the analysis device 1. Both the display unit 11 and the input unit 12 may include the same touch panel display. The communication unit 14 functions as a communication interface that establishes communication connections of various forms.

The storage unit 13 can be configured by a rewritable nonvolatile memory such as a hard disk and a flash memory. In addition to the program 132 stored in the storage unit 13, information defining the structure (architecture) of the model 131, parameters of the model 131 adjusted by learning processing to be described later, and learning data used for the learning processing are also stored.

The control unit 10 can include one or a plurality of processors such as a central processing unit (CPU) and a graphics processing unit (GPU), a read only memory (ROM), a random access memory (RAM), and the like. The control unit 10 reads and executes the program 132 in the storage unit 13 to virtually operate as an acquisition unit 10A, an output derivation unit 10B, a feature amount derivation unit 10C, and a learning unit 10D. The acquisition unit 10A acquires at least one of data of a microscopic image of the rubber composition and data processed based on the data via the input unit 12, the communication unit 14, and the like. The output derivation unit 10B inputs the data acquired by the acquisition unit 10A to the model 131, derives output data from the model 131, and generates a screen representing the output data. The feature amount derivation unit 10C derives one or a plurality of feature amounts relating to at least one of the structure and the physical properties of the rubber composition based on the output data. The learning unit 10D adjusts the parameters of the machine learning model by processing to be described later. It can be said that the parameters adjusted by learning are parameters that define the model 131.

### <3. Configuration of machine learning model>

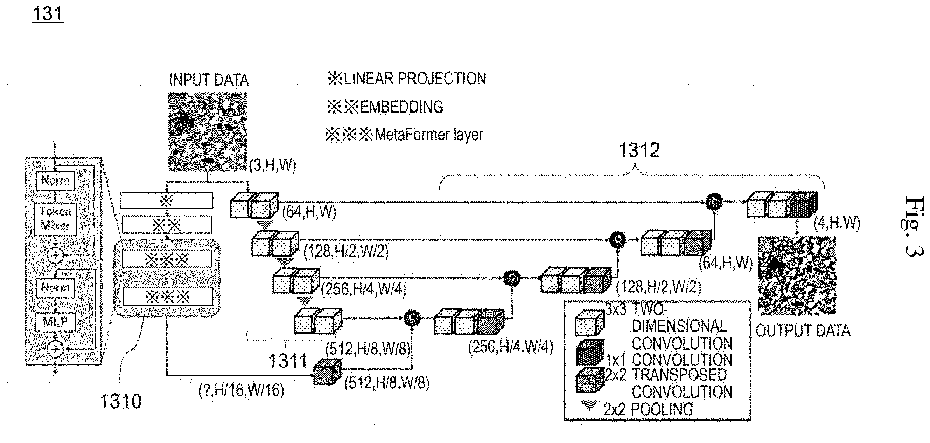

FIG. 3 is a diagram illustrating a structure of the model 131. The model 131 of the present embodiment is a deep learning model applied to image segmentation, particularly semantic segmentation, and more specifically, is a model based on a TransUNet model. The model 131 includes a Transformer encoder 1310 (Hereinafter, it is also simply referred to as a "T encoder 1310".) according to a Transformer model and a UNet encoder 1311 (Hereinafter, it is also simply referred to as a "U encoder 1311 ".) according to a UNet model. The model 131 further includes a UNet decoder 1312 (Hereinafter, it is also simply referred to as a "U decoder 1312".) according to the UNet model, and output data having the same size as the input data is output from the U decoder 1312.

The input data input to the model 131 is data of an image having a predetermined size defined by the number of pixels (H × W) in height × width. The input data is data generated based on data of a microscopic image obtained by image-capturing the rubber composition with a microscope, and may be data of an H × W microscopic image itself, or may be a plurality of patch images having the number of H × W pixels cut out from the microscopic image. Furthermore, the input data may be RGB image data or grayscale image data. As will be described later, the input data is input to the T encoder 1310 after being subjected to processing such as linear projection, and is also input to the U encoder 1311. That is, inputting input data to the model 131 includes inputting data generated based on the input data to the T encoder 1310 and inputting the input data to the U encoder 1311.

Note that the type of microscope for image-capturing the rubber composition is not particularly limited, but an electron microscope such as a transmission electron microscope or a scanning electron microscope is preferable, and a scanning electron microscope such as a backscattered electron microscope or a secondary electron microscope is more preferable. Furthermore, the secondary electron microscope mainly obtains information on silica, whereas the backscattered electron microscope obtains information on carbon, polymer, and silica. Therefore, the backscattered electron microscope is more preferable from the viewpoint of obtaining more information on the formulation. The magnification of the microscopic image is not particularly limited, but is preferably 2500 times to 100,000 times.

In a case where the input data to the model 131 is a series of patch images cut out from one image, the patch images are each converted into a one-dimensional vector before being input to the T encoder 1310, and then further linearly projected onto a lower-dimensional tensor. The matrix (filter) used for linear projection has parameters adjusted by learning processing to be described later. On the other hand, in a case where the input data to the model 131 is not a series of patch images, a series of patch images having a predetermined size may be generated based on the input data, each patch image may be converted into a one-dimensional vector, and then the vector may be linearly projected onto a lower-dimensional tensor. Furthermore, position information in the original image may be added to these linearly projected tensors. As described above, data (also referred to as a token) generated based on the input data is an input to the T encoder 1310 having a subsequent meta-structure (MetaFormer layer). The T encoder 1310 is an encoder that extracts an overall feature of the input data.

The T encoder 1310 is configured to extract features across a channel direction and a spatial direction by repeatedly mixing the channel direction and the spatial direction with respect to the input token. More specifically, normalization processing of the input token is performed in a Norm layer, and feature amounts corresponding to patches having different spatial positions are mixed in a subsequent TokenMixer layer. Furthermore, in another Norm layer, data normalization is performed, and feature amounts between different channels are mixed for each token by a subsequent multilayer perceptron (MLP). The number of MLP layers and the number of repetitions can be appropriately set. Furthermore, the T encoder 1310 has parameters adjusted by learning, such as a weighting coefficient and a bias of the MLP, and these parameters have been adjusted by learning processing to be described later. In the present embodiment, from the viewpoint of speeding up the processing and improving the accuracy of the region division, average pooling is adopted in the TokenMixer layer that performs feature extraction in the spatial direction, but the processing performed in the TokenMixer layer is not limited thereto.

The output from the T encoder 1310 is a feature amount representing a global feature of the input data. One transposed convolution layer is connected to the T encoder 1310, and the output of the T encoder 1310 is input to the transposed convolution layer. In the transposed convolution layer, a kernel having a predetermined size is applied, and a transposed convolution operation is performed on the input data. Parameters included in the kernel have been adjusted by learning processing to be described later. As a result, two-dimensional data (feature map) having an increased size with respect to the original data is output while retaining the features of the input data. This feature map has the same size as the output in the lowest hierarchy of the U encoder 1311 described later. The kernel size of the transposed convolution layer is not particularly limited, and can be appropriately set. Furthermore, an ReLU function is preferably applied at the time of the transposed convolution operation.

On the other hand, the U encoder 1311 has a structure in which two continuous two-dimensional convolution layers are connected by a pooling layer continuous to the convolution layers, thereby forming a plurality of hierarchies. Note that each hierarchy is skip-coupled to a corresponding hierarchy in the U decoder 1312 described later. In the convolution layer in each hierarchy of the U encoder 1311, feature extraction of the input image is performed. More specifically, two-dimensional data having a predetermined size is input to each convolution layer, and convolution by a convolution filter is performed. As a result, two-dimensional data (feature map) representing the features of the input image is output from each convolution layer. The convolution filter is a filter for extracting a feature of an input image, and parameters of the filter have been adjusted by learning processing to be described later. Furthermore, the ReLU function is preferably applied at the time of convolution operation, and thus, the extracted features are more emphasized. The number of filters in the convolution layer, a filter size, an interval of scanning the input data by the filter, a padding method of adjusting a size of the feature map to be created, and the like are not particularly limited, and can be appropriately set.

In each hierarchy, the feature map output from the second convolution layer is input to the pooling layer and down-sampled. As a result, a size of the two-dimensional data input to the convolution layer becomes smaller as the hierarchy goes down than a size of the feature map output in the upper hierarchy. In the pooling layer of the present embodiment, maximum value pooling is performed. That is, the input feature map is scanned by the kernel having a size smaller than that of the feature map, and the maximum value in the kernel is extracted. The size of the kernel and the interval of scanning the feature map can be appropriately set. In the present embodiment, the size of the kernel is 2×2, and the spatial resolution of the feature map is set to be half. Note that the pooling layer does not have parameters adjusted by learning.

In the U encoder 1311, a feature map of a small size in which smaller features are captured is output each time the processing by the combination of the convolution layer and the pooling layer progresses. The feature maps output in the last hierarchy of the U encoder 1311 are the same number and have the same size as the feature maps output from the transposed convolution layers connected after the T encoder 1310. Data obtained by combining these feature maps (Hereinafter, also referred to as a "combination map") is an input to the U decoder 1312. By inputting the combination map created in this manner to the U decoder 1312, region division reflecting both global information and strong local information in the input data becomes possible.

The U decoder 1312 has a structure in which hierarchies that are skip-coupled to the respective hierarchy of the U encoder 1311 are formed, and in each hierarchy, one transposed convolution layer is connected after two continuous two-dimensional convolution layers. The processing performed in the two-dimensional convolution layer and the transposed convolution layer is as described above. However, in the uppermost hierarchy in which the output data of the final model 131 is output, not the transposed convolution layer but one 1×1 convolution layer is connected after the two continuous two-dimensional convolution layers.

The above-described combination map is input to the first convolution layer of the lowermost hierarchy of the U decoder 1312. Then, by the processing in the subsequent convolution layers and transposed convolution layer, two-dimensional data having a larger size than the combination map is generated while the features of the input combination map are maintained.

The two-dimensional data output from the transposed convolution layer of the lowermost hierarchy is passed to the first convolution layer of a hierarchy one level up together with the two-dimensional data (feature map) output from the convolution layer of the U encoder 1311 to which the skip coupling is performed. As a result, it is possible to generate two-dimensional data larger than the passed two-dimensional data while holding the position information of the local feature extracted by the U encoder 1311. In the U decoder 1312, this processing is repeated for each hierarchy, and the size of the output two-dimensional data approaches the size of the input data of the model 131 each time this processing is repeated.

As described above, data to be the output of the model 131 is finally output from the 1×1 convolution layer in the uppermost hierarchy of the U decoder 1312. This output data is data of an image in which different brightness are assigned to a region occupied by a texture of each formulation in the input data of the model 131. That is, the output data is data defining a region appearing corresponding to the formulation of the rubber composition in the microscopic image of the rubber composition, and can also be said to be data in which each pixel is labeled to represent the formulation corresponding to the pixel. Note that the number of labels is not particularly limited, and can be appropriately set at the time of learning the model 131.

### <4. Analysis method>

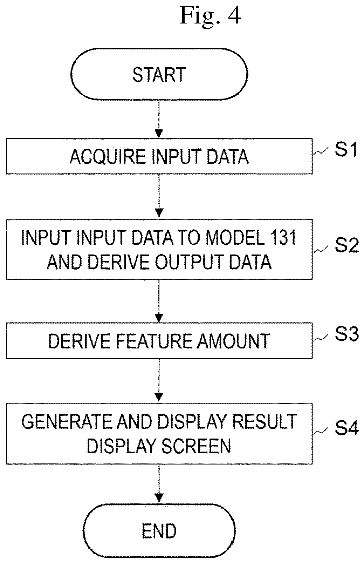

FIG. 4 is a flowchart showing a flow of an analysis method including region division processing of a microscopic image of a rubber composition and derivation processing of a feature amount based on a result of the region division executed by the analysis device 1. Hereinafter, an operation of the analysis device 1 will be described with reference to FIG. 4.

First, the acquisition unit 10A acquires input data (step S1). As described above, the input data is data generated from a microscopic image obtained by image-capturing the rubber composition with a microscope. The acquisition of the input data by the acquisition unit 10A may be performed via a recording medium such as a CD-ROM or a USB memory, or may be performed by reading data held by an external device via network communication. In a case where the input data is a patch image cut out from a microscopic image of the rubber composition, the acquisition unit 10A may first acquire a microscopic image of the rubber composition and cut out the microscopic image to create a patch image.

Subsequently, the output derivation unit 10B inputs the input data acquired in step S1 to the model 131 and derives output data from the model 131 (step S2). As described above, the output is data defining a region that appears corresponding to the formulation of the rubber composition, and is data in which the region is divided for each formulation with respect to the input data.

Subsequently, the feature amount derivation unit 10C derives a feature amount related to at least one of a structure and physical properties of the rubber composition based on the output data output in step S2 (step S3). A method of deriving the feature amount is not particularly limited, but in the present embodiment, the feature amount is derived by a method using persistent homology (Hereinafter, also referred to as "PH method"). As described below, in the present embodiment, the PH method is used to derive the feature amount regarding a dispersion state of a texture formed by a specific formulation, a shape of the texture formed by the formulation, and a size of the texture formed by the formulation.

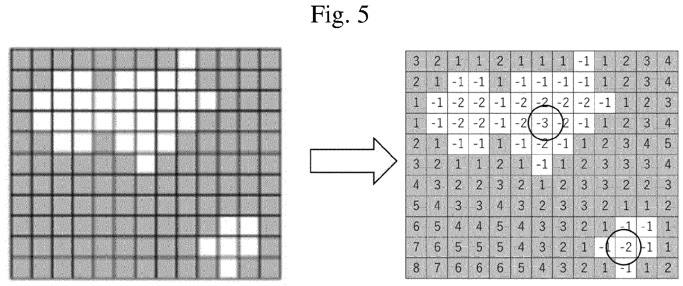

FIG. 5 is a diagram illustrating a principle of deriving a feature amount by the PH method. First, the feature amount derivation unit 10C creates a binarized image for distinguishing a region corresponding to a target formulation of interest from other regions based on the region-divided image output in step S2. In the example illustrated on the left of FIG. 5, pixels in the region corresponding to the formulation of interest are represented in white and pixels in the other regions in gray. Thereby, a texture of the formulation of interest in the entire image is represented as an island structure.

Subsequently, the feature amount derivation unit 10C sets the pixels constituting an outline of the island structure as -1 pixels, allocates a value obtained by subtracting 1 from the value of the adjacent pixel as a distance from these pixels increases by one pixel toward the inside of the island structure, and allocates a value of -1 or less to all the pixels of the island structure. Specifically, the feature amount derivation unit 10C allocates -1 to the pixels located on an outermost side of the island structure, allocates -2 to the pixels inside the island structure adjacent to the -1 pixels, and allocates -3 to the pixel inside the island structure adjacent to the -2 pixels. In this way, when the value representing a Manhattan distance is allocated to all the pixels forming the island structure, the pixel having the smallest allocated value is determined as a center of the island structure. In the example illustrated on the right side of FIG. 5, a -3 pixel having an island structure at the upper left of the binarized image and a -2 pixel having an island structure at the lower right of the binarized image are determined as central pixels of the respective island structures.

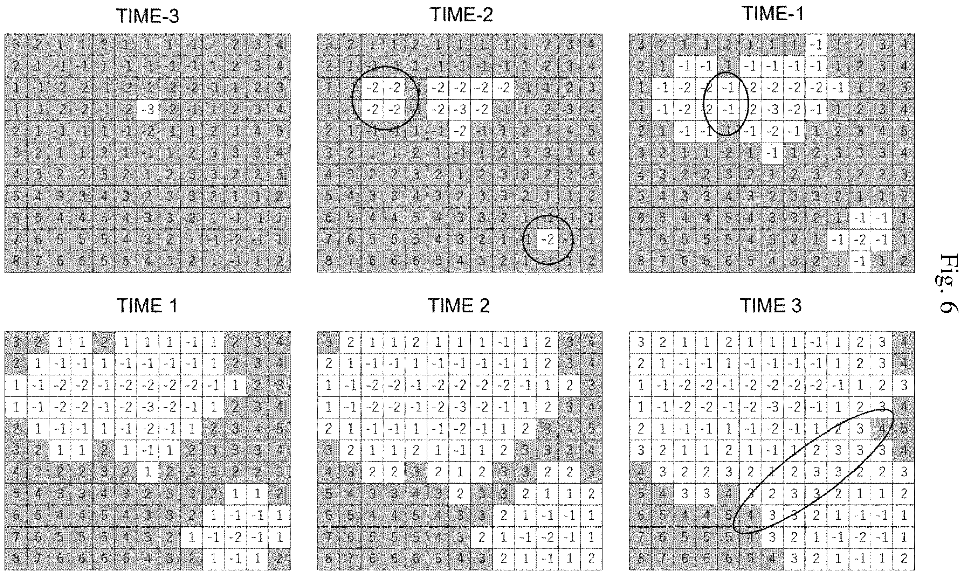

Next, the feature amount derivation unit 10C converts into a histogram a process of generating or combining a white region when the white region is expanded by one pixel per unit time based on a pixel having the smallest value in the binarized image. This algorithm will be described with reference to FIG. 6 as an example. First, time (-3) corresponding to a value of a reference pixel is set as generation time of a center of the island structure, and at this time, it is assumed that only a -3 pixel is white and the other pixels are gray. At the next time (-2), a white region corresponding to one pixel from the reference pixel is expanded, and -2 pixels are newly set to white. As a result, two new island structures are generated in addition to the island structure including the reference pixel. The time (-2) at which the island structures are newly generated is defined as a generation time. In the same manner, the white region is further expanded by one pixel at the next time (-1). As a result, two independent island structures at the time (-2) are bonded to form one island structure. At the next time (1), a white region for one pixel is expanded outside the original island structures. In FIG. 6, 1 is displayed in a pixel that has newly become a white region. Similarly, at the next time (2), a white region is further expanded by one pixel from the pixel in which 1 is displayed. In FIG. 6, 2 is displayed in the pixel that has newly become the white region. At the next time (3), the white region is further expanded by one pixel from the pixel in which 2 is displayed. As a result, the originally independent two island structures are bonded to form one island structure.

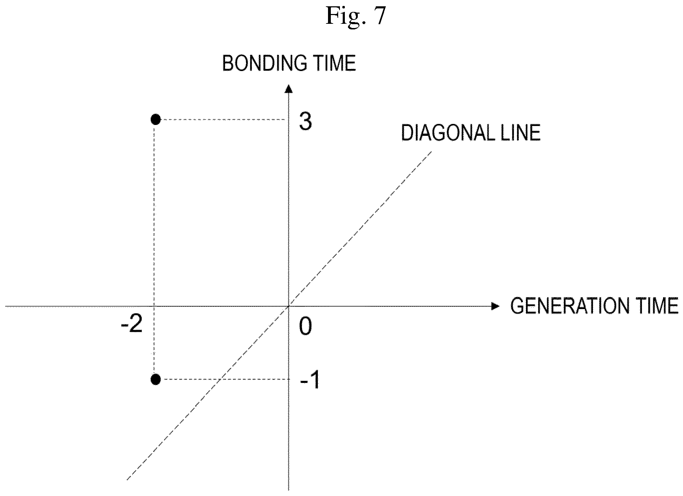

As illustrated in FIG. 7, a two-dimensional histogram is generated by plotting, on a plot plane in which a horizontal axis represents a generation time and a vertical axis represents a bonding time, the generation time at which an island structure is newly generated by the above algorithm and the bonding time at which two independent island structures are bonded. In the example of FIG. 7, since the bonding times are -1 and 3 with respect to the generation time -2, two points (-2, -1) and (-2, 3) are plotted on the plot plane. In the present embodiment, this histogram is vectorized and the vectorized histogram is the feature amount related to the structure of the rubber composition.

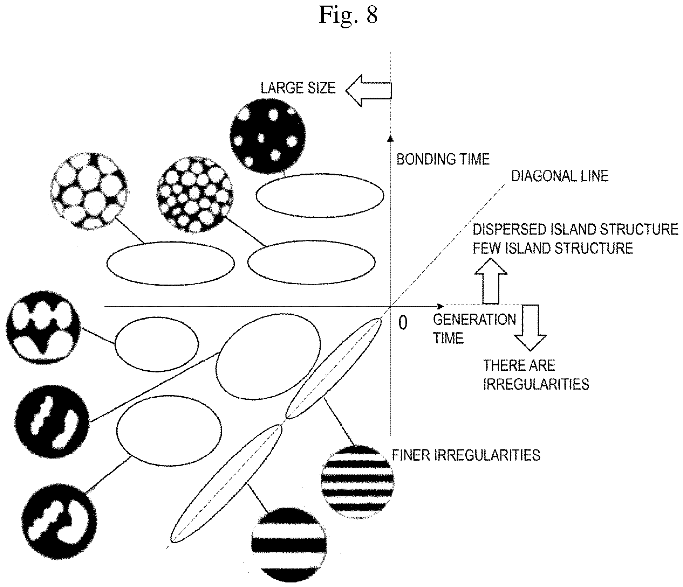

FIG. 8 is a diagram illustrating information represented by positions of points plotted on the two-dimensional histogram. As illustrated in FIG. 8, the farther the plot point is from the horizontal axis in an upward direction, the less island structure is formed by the formulation of interest, or the greater a degree of dispersion of the formulation of interest. This is because when the island structures are further separated from each other, a longer time is required until the two island structures are bonded in the above algorithm. On the other hand, plotted points below the horizontal axis indicate that a texture formed by the formulation of interest has irregularities. This is because in this case, it is indicated that the originally single island structure is generated by the bonding of two or more island structures in the above algorithm. Furthermore, the further the plot point is left from the vertical axis, the larger the size of the island structure formed by the formulation of interest. This is because when the size of the island structure is large, the time at which the island structure is newly generated in the algorithm is a time earlier than the time (-1) at which the outline of the original island structure is reached. Moreover, the closer the plot point is to a diagonal line of the plot plane, the more fine the irregularities in the texture formed by the formulation of interest. This is because when the irregularities of the texture are fine, a difference between the generation time and the bonding time of the island structure decreases in the algorithm. Note that when the shape of the originally single island structure has many irregularities and many constrictions, the number of plot points increases near the bonding time (-1). The feature amount derivation unit 10C derives a feature amount related to the structure of the rubber composition as described above.

In a case where the correlation between the structure of the specific formulation and the physical properties of the rubber composition is known, the feature amount derivation unit 10C can further derive the feature amount related to the physical properties of the rubber composition from the feature amount related to the structure of the rubber composition. According to the study of the present inventors, it has been found that the feature amount related to at least one of the dispersion state, the shape and the size of the aggregate for a specific formulation has a correlation with physical properties of the rubber composition such as Mooney viscosity, type A hardness (Hs), Swell (toluene swelling index), specific gravity, M100 (modulus at 100% elongation), M200 (modulus at 200% elongation), M300 (modulus at 300% elongation), TB (tensile strength), EB (elongation), E* (storage elastic modulus, 0 °C, 0.25%), tanδ (loss elastic modulus, 0°C, 2.5%), E* (30°C, 1%), tanδ (30°C, 1%), TD (temperature dispersion), Tg (glass transition temperature), TDtanδ, and TD 1/2 W. Note that TDtanδ is calculated based on the temperature dispersion, and TD 1/2 W represents a half-value width of a waveform of a temperature dispersion curve. As described above, in a case where the correlation between at least one of the feature amounts for a specific formulation and specific physical properties is found, it can be said that deriving the feature amount is also deriving the feature amount related to the physical properties of the rubber composition.

After step S3, the feature amount derivation unit 10C may generate a result display screen indicating the derived feature amount and cause the display unit 11 to display the result display screen (step S4). The result display screen may include output data by the model 131.

### <5. Learning method>

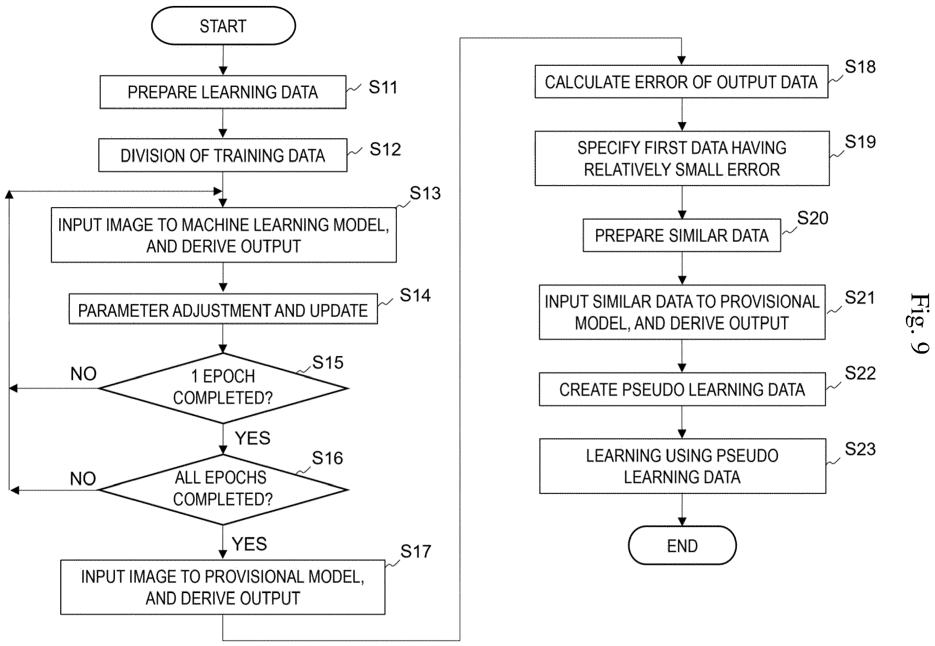

FIG. 9 is a flowchart showing a flow of a learning method of the machine learning model executed by the generation device 1, that is, a generation method for the model 131. Hereinafter, a learning method of a model having the structure illustrated in FIG. 3 will be described with reference to FIG. 9.

First, learning data for learning the machine learning model is prepared (step S11). The learning data is a plurality of pieces of data in which data (hereinafter, also referred to as "first data") generated from a microscopic image obtained by image-capturing a rubber composition with a microscope and correct answer data (second data) are combined. The first data may be a microscopic image itself having a prescribed size of H × W, or may be a patch image having a prescribed size of H × W cut out from the microscopic image. The microscopic image includes microscopic images of a plurality of rubber compositions different in formulation (at least one of the type and formulation ratio of the formulation), kneading conditions, and the like. In the present embodiment, the magnification ratio of the microscope of the microscopic image is constant regardless of the image, but the magnification ratio of the microscope may be different depending on the image. Furthermore, for example, the same rubber composition may also include a plurality of microscopic images having different magnification ratios and image-capturing ranges of the microscope. In addition, the microscope for image-capturing the rubber composition is as described in the description of the input data to the model 131.

The correct answer data is data defining each region that appears in the first data corresponding to each formulation of the rubber composition. In other words, the correct answer data is data obtained by labeling (annotating) all the pixels of the first data to indicate a region occupied by a texture of which formulation the pixel belongs to. The number of labels can be the number of types of formulations of the rubber composition that appear in the microscopic image. The annotation can be performed by a known method, but the region division of the first data as a reference of the annotation does not make the brightness of boundary pixels between different island structures gradation, and assigns a label corresponding to some formulation to these pixels. Therefore, it is preferable that the annotation reflects labeling by a person skilled in observing the rubber composition. As a result, the correct answer data conforms to more conventional knowledge.

In the present embodiment, the prepared learning data is taken into the analysis device 1 via a storage medium or a network, and stored in the storage unit 13 as learning data 134 by the learning unit 10D. The learning unit 10D stores the learning data 134 separately in advance into training data for parameter adjustment and test data for accuracy verification. A ratio between the two can be appropriately set.

Subsequently, the learning unit 10D divides the training data into a predetermined number of pieces of data to form a plurality of subsets (step S12). The predetermined number is the number of pieces of data input to the machine learning model per one time in the next step S13, and can be appropriately set.

Subsequently, the learning unit 10D selects one of the subsets, inputs the first data included in the selected subset to the machine learning model, and derives output data from the machine learning model (step S13). The output data is data corresponding to the correct answer data combined with the input first data, and in the present embodiment, is data in which a region corresponding to the formulation is defined. Note that, in step S13, in each convolution layer, batch normalization processing may be introduced after the convolution operation and before the ReLU function is applied. As a result, the learning can be stabilized and the speed can be improved.

Subsequently, the learning unit 10D adjusts the parameters so that a value of an error function between the output derived in step S13 and the correct answer data combined with the first data input in step S13 is minimized (step S14). Examples of the error function include a cross entropy error function and the like. The learning unit 10D adjusts and updates the parameters of the linear projection matrix of the machine learning model, the T encoder, the bias in each convolution layer, the convolution filter, and the like according to, for example, a stochastic gradient descent method.

Subsequently, the learning unit 10D determines whether or not learning of one epoch has been completed (step S15). In the present embodiment, for each subset created in step S12, in a case where steps S13 and S14 make one round, it is determined that the learning of one epoch is completed. In a case where it is determined that the learning of one epoch is not completed (NO), the learning unit 10D repeats steps S13 to S14 using a subset that is not yet used. On the other hand, in a case where it is determined that the learning of one epoch has been completed (YES), step S16 is executed.

In step S16, the learning unit 10D determines whether or not learning of all epochs is completed. The total number of epochs is not particularly limited, and can be appropriately set. In a case where it is determined that the learning of all epochs is not completed (NO), the learning unit 10D repeats steps S13 to S15 while selecting subsets in the same order as the previous epoch. On the other hand, in a case where it is determined that the learning of all epochs has been completed (YES), the learning unit 10D stores the latest parameters in the storage unit 13. That is, the model 131 in which supervised learning is completed is generated by the above procedure. However, in the present embodiment, in order to increase the accuracy of the output data of the model 131, the learning unit 10D further executes semi-supervised learning. Therefore, hereinafter, a model after the completion of the supervised learning and before the completion of the semi-supervised learning may be distinguished and referred to as a "provisional model".

Subsequently, the learning unit 10D derives output data corresponding to the correct answer data combined with the input first data from the provisional model by inputting the first data of the verification data to the provisional model (step S17).

Subsequently, the learning unit 10D calculates an error of the output data derived in step S17 with respect to the correct answer data (step S18). The method of calculating the error is not particularly limited, and can be performed according to a known method.

Subsequently, the learning unit 10D specifies the first data included in the verification data with a relatively small error calculated in step S18 (step S19). The learning unit 10D may specify the first data of the verification data in which based on a predetermined threshold, the calculated error is less than or equal to the threshold or less than the threshold. Alternatively, the first data having a relatively small error may be specified among the output data derived from the provisional model in step S16. Note that the first data specified here is preferably data for which appropriate region division by the provisional model is considered to be relatively difficult. The accuracy of the model 131 can be further improved by setting similar data to be described later to data similar to the first data in which an error from the correct answer data is relatively small while the difficulty of region division is relatively high for the provisional model.

Next, similar data having the same size as the first data specified in step S19 and having a high degree of similarity to the first data is prepared (step S20). The similar data may be, for example, data generated from a microscopic image of the rubber composition that is not included in the learning data prepared in step S11. A method of determining a degree of similarity to the first data is not particularly limited, and for example, data having a brightness distribution closer to that of the first data can be prepared as similar data. Unlike the first data, the similar data is not combined with the correct answer data. The learning unit 10D takes similar data into the analysis device 1 via a storage medium or a network, and stores the similar data in the storage unit 13.

Subsequently, the learning unit 10D derives output data from the provisional model by inputting the similar data to the provisional model (step S21).

Subsequently, the learning unit 10D sets the derived output data as pseudo correct answer data and combines the pseudo correct answer data with the original similar data to create pseudo learning data (step S22). The created pseudo learning data is stored in the storage unit 13 similarly to the learning data.

The learning unit 10D further continues learning of the provisional model by using the pseudo learning data created in step S22 (step S23). That is, in the following processing, processing similar to steps S12 to S16 is performed on the combined data of the pseudo learning data and the learning data, and the parameters of the provisional model are further adjusted. As a result, it is possible to generate the highly accurate model 131 corresponding to the input data having a higher degree of difficulty in region division than the provisional model. Note that the learning of the provisional model may be ended without waiting for completion of the learning of all epochs in a case where the error is reduced to some extent and can be regarded as stable.

### <6. Features>

In the analysis device 1 of the above embodiment, the region division of the input microscopic image is performed by the model 131 that has learned the relationship between the microscopic image of the rubber composition and the region appearing on the microscopic image. Since the model 131 has been trained using the learning data labeled according to the actual state of the rubber composition, it is possible to perform region division with higher reliability as compared with region division by a conventional image processing algorithm. Therefore, a highly reliable feature amount can be extracted based on the output data of the model 131, and the estimation accuracy of the structure and physical properties of the rubber composition based on the microscopic image is improved. In addition, it is expected that new findings will be obtained on the correlation between the features appearing in the microscopic image and the structure and physical properties of the rubber composition.

### <7. Modification examples>

Although one embodiment of the present invention has been described above, the present invention is not limited to the above embodiment, and various modifications can be made without departing from the gist thereof. The gist of the following modification examples can be appropriately combined.
(1) In the above embodiment, a model based on the TransUNet model is used as the model 131, but the structure of the model 131 is not limited thereto. The model 131 may be, for example, a model based on an attention network, SegNet, a full-layer convolutional network (FCN), a feature pyramid network, PSPNet, RefineNet, a recurrent neural network (RNN)-based model, UNet, USegnet, TransNet, TransUNet, Large Kernel Matters, Deeplabv3+, or the like, which is a model belonging to a category of semantic segmentation, or a model obtained by appropriately combining these. Furthermore, a model other than the above category, for example, a model based on ResNet, a support vector-machine (SVM), a neural network (NN) model, a K-NN model, clustering, k-means, a decision tree, or a logistic regression model, a model obtained by appropriately combining these, or the like may be used. Furthermore, the layer configuration of the model 131 is not limited to that of the above embodiment. For example, the configuration of the T encoder 1310 may be appropriately changed, the convolution layers of the U encoder 1311 and the U decoder 1312 may be added or omitted, and these layers may be increased or decreased.
(2) The learning method according to the above embodiment is merely an example, and is not limited to the method of the above embodiment. For example, the error calculation method between the output data and the correct answer data and the parameter adjustment algorithm can be appropriately changed.
(3) In the above embodiment, the analysis device 1 is configured as one device, but the functions of the units 10A to 10D and the storage unit 13 may be distributed to a plurality of devices. Therefore, each step of the analysis method and the generation method for a trained model may be executed in a distributed manner by one or a plurality of computers.
(4) In addition to the CPU and the GPU, the control unit 10 of the analysis device 1 may include a vector processor, a field programmable gate array (FPGA), an application specific integrated circuit (ASIC), other artificial intelligence dedicated chips, and the like. Furthermore, the operation of the control unit 10 may be executed by one or a plurality of processors.
(5) The method of deriving the feature amount related to the structure and physical properties of the rubber composition based on the output data of the model 131 is not limited to the method of the above embodiment, and may be performed by a method other than the PH method. For example, based on the output data of the model 131, an average size of regions corresponding to a specific formulation, a distance between the regions, an index indicating a shape of the region, and an index relating to physical properties correlated therewith can be derived by a known image processing algorithm, and these indexes are also included in the feature amount of the present invention. Furthermore, a new machine learning model that learns the relationship between the output data of the model 131 and the feature amount regarding at least one of the structure and the physical properties of the rubber composition may be constructed, and based on an output data of this machine learning model, the feature amount regarding at least one of the structure and the physical properties of the rubber composition may be derived.

### [Examples]

Hereinafter, examples of the present invention will be described in detail. However, the present invention is not limited to these examples.

### <Experiment 1>

Learning data in which input data generated from microscopic images of a plurality of rubber compositions having different formulations and correct answer data were combined was prepared, and divided into training data and verification data. Using the training data, Model 1 having the structure illustrated in FIG. 3 and Model 2 according to the known UNet model were trained. Note that learning using the pseudo learning data of the above embodiment was applied to learning of Model 1, and this was repeated 20 times. Formulations of the rubber compositions were Formulation 01, Formulation 02, Formulation 03, Formulation 04 and others, and the labels attached to the correct answer data were four types corresponding to carbon (CB), styrene-butadiene rubber (SBR), natural rubber (NR), and silica (Si). Image data of verification data was input to each of trained Models 1 and 2, and output data was derived. For the derived output data, an F value was calculated for each formulation and label. The F value is an index calculated by a harmonic average of a matching rate and a reproduction rate, and it may be said that the accuracy of the model is higher as the F value is closer to 1.

### <Result 1>

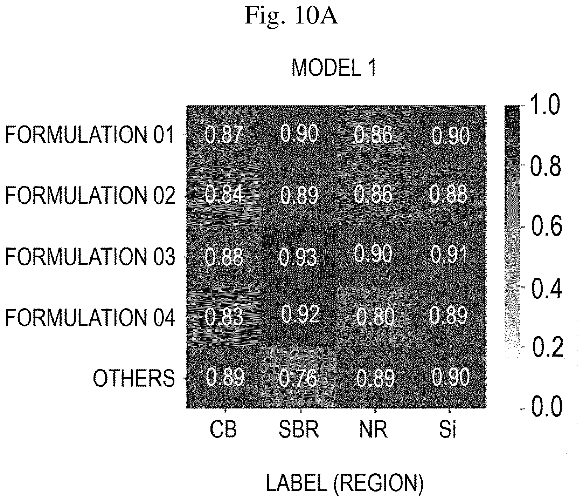

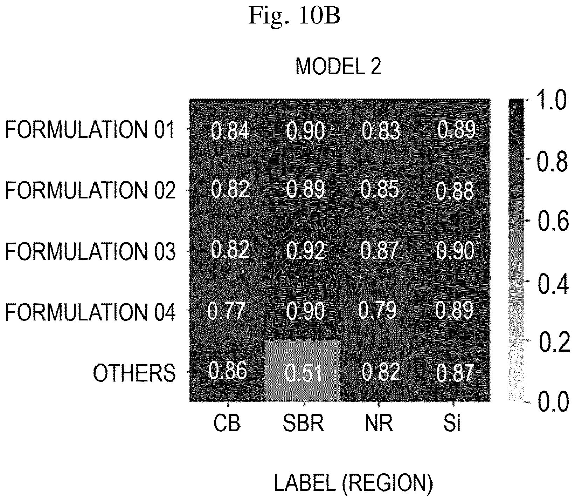

For Models 1 and 2, the F values calculated for each formulation and label were the results illustrated in FIGS. 10A and 10B, respectively. As illustrated in FIG. 10A, in Model 1, it was confirmed that the unevenness of the F value with respect to the formulation and the type of label was relatively small. Furthermore, an average of all F values of Model 1 was 0.887, and it was confirmed that the region division was performed with sufficient accuracy under any conditions. On the other hand, an average of all the F values of Model 2 was 0.856, and it was confirmed that the accuracy of region division as a whole was sufficient. However, as illustrated in FIG. 10B, in Model 2, it was confirmed that the F value for the SBR in the other formulation was smaller than the F values for the other conditions, and the unevenness of the F value due to the conditions was larger than that of Model 1.

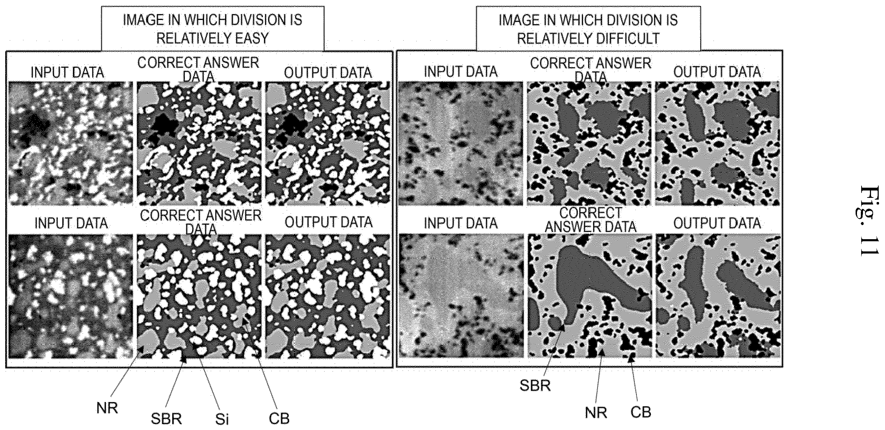

FIG. 11 is a diagram for comparing input data input to Model 1 with correct answer data combined therewith, and output data from Model 1 with respect to the input data. From FIG. 11, it has been observed that the region division is satisfactorily performed so as to be close to the correct answer data in Model 1. Furthermore, for reference, an example of input data for which region division is relatively easy is illustrated on the left side of FIG. 11, and an example of input data for which region division is relatively difficult is illustrated on the right side. As can be seen, in the input data for which the region division is relatively difficult, a contrast of the pixel is relatively small. Note that, in a part of the correct answer data, the attached label is shown as the name of the formulation, but the correspondence between the brightness of the pixel and the formulation is not limited thereto.

### <Experiment 2>

A plurality of input data generated from microscopic images of rubber compositions having different formulations were input to Model 1 trained in Experiment 1, and a plurality of output data were derived. Based on the output data, a histogram of NR and a histogram of Si by the PH method of the above embodiment were created for each formulation, and based on this, a feature amount related to the structure of NR and a feature amount related to the structure of Si were derived, respectively. The formulations of the rubber compositions were common to those in Experiment 1: Formulation 01, Formulation 02, Formulation 03, and Formulation 04. According to the conventional knowledge, in Formulation 01 and Formulation 02, the size of the island structure of NR is relatively small, the shape has many undulations (irregularities), and there is a tendency that the distances are close to each other. On the other hand, in Formulation 03 and Formulation 04, the size of the island structure of NR is relatively large, the shape has few undulations and is rounded, and there is a tendency that the distances are far from each other. The derived feature amounts related to the structure of NR were classified into two categories of Formulations 01 and 02 or Formulations 03 and 04 using a logistic regression model, and it was verified whether or not feature extraction conforming to the conventional knowledge could be performed. In addition, according to the conventional knowledge, in Formulations 01 and 03, a size of the Si aggregate is relatively small, and in Formulations 02 and 04, a size of the Si aggregate is relatively large. The derived feature amounts related to the structure of Si were classified into two categories of Formulations 01 and 03 or Formulations 02 and 04 using the logistic regression model, and it was verified whether or not feature extraction conforming to the conventional knowledge could be performed.

### <Result 2>

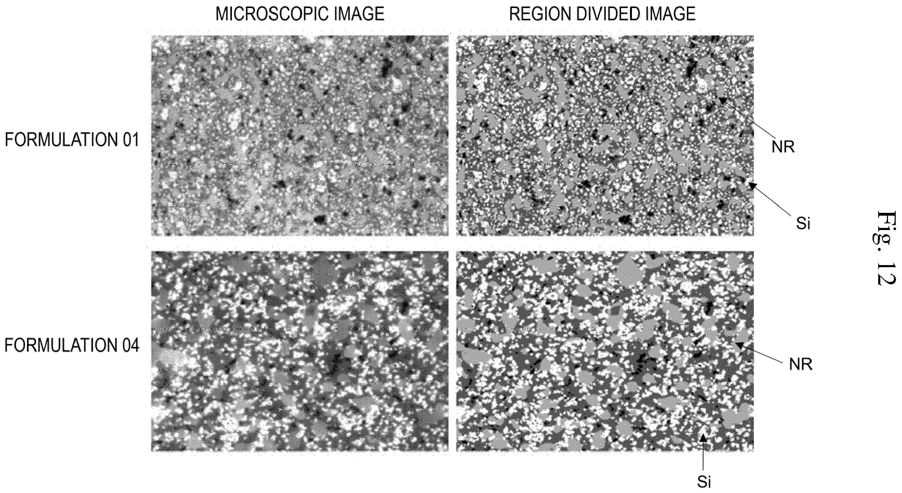

A correct answer rate of classification of the feature amount related to the structure of NR by the logistic regression model was 96%, and a correct answer rate of classification of the feature amount related to the structure of Si by the logistic regression model was 99%. This confirmed that feature extraction concerning the structure of the rubber composition that is consistent with the conventional knowledge can be performed based on the output data of Model 1. For reference, for the rubber compositions of Formulations 01 and 04, FIG. 12 illustrates a diagram comparing microscopic images of the rubber compositions and images resulting from region division using Model 1 on the microscopic images. From FIG. 12, it can be confirmed that the size of the Si aggregate tends to be small and the undulation tends to occur in the island structure of NR in Formulation 01, whereas the size of the Si aggregate tends to be large and the island structure of NR tends to be rounded in Formulation 04. These tendencies are reflected in the images obtained as a result of performing the region division by Model 1.

### <Experiment 3>

With respect to the output data derived in Experiment 2, a histogram of Si by the PH method of the above embodiment was created, and a feature amount regarding the structure of Si was derived based on the histogram. The correlations between the derived feature amount and the physical properties Za, Zb, Zc, Zd, X1 to X5, and Y1 to Y6 of the rubber composition were examined. More specifically, correlation analysis between each element in the feature amount (vector) based on the histogram of Si and each physical property was performed, a correlation coefficient from -1 to 1 was calculated for each element, and a correlation between the feature amount and the physical property was confirmed based on the correlation coefficient.

### <Result 3>

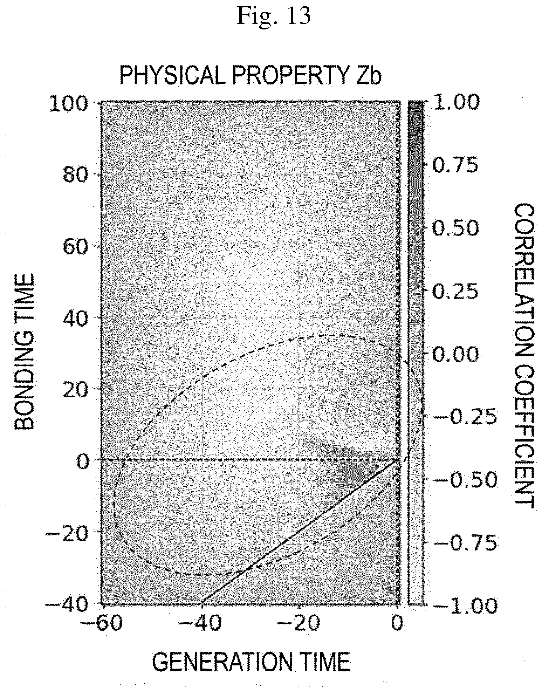

As a result of the examination, it was confirmed that the physical properties Za, Zb, and Zc have a forward correlation with those in which the shape of the Si aggregate has undulation and those in which the aggregate is rounded and has a large size. FIG. 13 is an example of a diagram visualizing how correlation coefficients are distributed on a histogram of Si, and illustrates a result of examination on the physical property Zb. Furthermore, it was confirmed that the physical property Zd has a forward correlation with Si aggregates having roundness and a relatively small distance between aggregates, and has a reverse correlation tendency with the physical properties Za, Zb, and Zc. Note that the physical property Za is Mooney viscosity, Zb is type A hardness (Hs), Zc is Swell, and Zd is specific gravity. Similarly, it was confirmed that the physical properties X1 to X3 have a forward correlation with those in which the shape of the aggregates of Si has undulation, those in which the aggregates are rounded and have a large size, and those in which the aggregates are rounded and the distance between the aggregates is relatively large. It was confirmed that the physical properties X4 and X5 have a forward correlation with those in which the shape of the Si aggregate has undulation and those in which the aggregate is rounded and the distance between the aggregates is relatively small. Note that the physical property X1 is M100, X2 is M200, X3 is M300, X4 is TB, and X5 is EB. Moreover, it was confirmed that the physical properties Y1 to Y5 have a forward correlation with those in which the shape of the Si aggregate has undulation and those in which the aggregate is rounded and has a large size. On the other hand, it was confirmed that the physical property Y7 has a forward correlation with Si aggregates having roundness, a small size, and a relatively small distance between aggregates, and has a reverse correlation tendency to the physical properties Y1 to Y5. It was confirmed that the physical property Y6 has a forward correlation with Si aggregates having roundness and a relatively large distance between aggregates. Note that physical property Y1 is E* (0°C, 0.25%), Y2 is tanδ (0°C, 2.5%), Y3 is E* (30°C, 1%), Y4 is tanδ (30°C, 1%), Y5 is TD, Tg, Y6 is TDtanδ, and Y7 is TD 1/2 W. From the above results, it was confirmed that one or a plurality of feature amounts relating to the physical properties of the rubber composition can be derived based on the output data output from the model in the embodiment.

### Reference Signs List

- 1: Analysis device
- 10: Control unit
- 10A: Acquisition unit
- 10B: Output derivation unit
- 10C: Feature amount derivation unit
- 10D: Learning unit
- 131: Model

## Claims

1. An analysis method for a rubber composition, the analysis method comprising:
acquiring input data generated from a microscopic image obtained by image-capturing a rubber composition with a microscope, the microscopic image showing a formulation contained in the rubber composition;
inputting the input data to a trained machine learning model; and
deriving output data from the trained machine learning model,
wherein the output data is data defining a region that appears in the microscopic image corresponding to the formulation.

2. The analysis method according to claim 1, further comprising deriving a feature amount related to at least one of a structure and a physical property of the rubber composition based on the output data.

3. The analysis method according to claim 1 or 2, wherein the trained machine learning model is configured as a segmentation model.

4. The analysis method according to claim 3, wherein the trained machine learning model is configured as a model selected from a group consisting of an attention network, SegNet, a feature pyramid network, UNet, PSPNet, TransNet, and TransUNet, or a model based on a model selected from the group.

5. The analysis method according to claim 4, wherein the trained machine learning model is configured as a model including a Transformer encoder and a UNet encoder.

6. The analysis method according to claim 5, wherein inputting the input data to the trained machine learning model includes inputting data based on the input data to the Transformer encoder and inputting the input data to the UNet encoder.

7. The analysis method according to any of claims 1 to 6, wherein the microscope is a backscattered electron microscope.

8. The analysis method according to any of claims 2 to 7, wherein the feature amount relates to at least one of a dispersion state of the formulation, a shape of texture formed by the formulation, and a size of texture formed by the formulation.

9. The analysis method according to any of claims 2 to 7, wherein the feature amount relates to at least one of viscosity, hardness, toluene swelling index, specific gravity, glass transition temperature (Tg), temperature dispersion (TD), modulus, tensile strength, elongation, storage elastic modulus, and loss elastic modulus of the rubber composition.

10. A generation method for a trained model, the generation method comprising:
preparing learning data in which a plurality of pieces of first data generated from a microscopic image obtained by image-capturing a rubber composition with a microscope, the microscopic image showing a formulation contained in the rubber composition, and a plurality of pieces of second data defining a region appearing in the microscopic image corresponding to the formulation are combined;
dividing the learning data into training data and verification data; and
adjusting a parameter defining a machine learning model such that when data corresponding to the first data is input using the training data, data corresponding to the second data is output.

11. The generation method for a trained model according to claim 10, further comprising:
deriving output data corresponding to the second data by inputting first data included in the verification data to the machine learning model in which the parameter is adjusted;
calculating an error of the output data with respect to the second data; and
specifying the first data included in the verification data in which the error is relatively small.

12. The generation method for a trained model according to claim 10 or 11, further comprising:
newly preparing similar data having a high degree of similarity to the specified first data;
deriving output data by inputting the similar data to the machine learning model in which the parameter is adjusted;
using the derived output data as pseudo correct answer data, creating pseudo learning data in which the similar data and the pseudo correct answer data are combined; and
further adjusting the parameter such that when the similar data is input to the machine learning model in which the parameter is adjusted, data corresponding to the pseudo correct answer data is output.

13. An analysis device (1) comprising one or a plurality of processors configured to execute the analysis method according to any of claims 1 to 9.

14. A generation device (1) for a trained model, the generation device comprising one or plurality of processors configured to execute the generation method for a trained model according to any of claims 10 to 12.

15. An analysis program (132) causing one or plurality of processors to execute the analysis method according to any of claims 1 to 9.

16. A generation program (132) for a trained model, the generation program causing one or plurality of processors to execute the generation method for a trained model according to any of claims 10 to 12.
